(19) Europäisches Patentamt
European Patent Office
Office européen des brevets

(11) **EP 2 543 998 B1**

(12) **EUROPÄISCHE PATENTSCHRIFT**

(45) Veröffentlichungstag und Bekanntmachung des Hinweises auf die Patenterteilung:
**29.10.2014 Patentblatt 2014/44**

(51) Int Cl.:
*G01N 33/49* *(2006.01)*    *G01N 11/04* *(2006.01)*

(21) Anmeldenummer: **11172972.9**

(22) Anmeldetag: **07.07.2011**

(54) **Verfahren zur Standardisierung von Messergebnissen in einem System zur Messung der Thrombozytenfunktion**

Method for standardising measurement results in a system for measuring thrombocyte function

Procédé de standardisation de résultats de mesure dans un système de mesure de la fonction des thrombocytes

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**09.01.2013 Patentblatt 2013/02**

(73) Patentinhaber: **Siemens Healthcare Diagnostics Products GmbH**
**35041 Marburg (DE)**

(72) Erfinder: **Rechner, Andreas**
**35041 Marburg (DE)**

(56) Entgegenhaltungen:
**EP-A1- 1 850 135**

• **A. R. RECHNER: "Platelet function testing in clinical diagnostics", HÄMOSTASEOLOGIE, Bd. 31, Nr. 2, 9. Dezember 2010 (2010-12-09), Seiten 79-87, XP55012133, ISSN: 0720-9355, DOI: 10.5482/ha-1133**
• **BOCK ET AL: "Standardization of the PFA-100R platelet function test in 105 mmol/l buffered citrate: effect of gender, smoking, and oral contraceptives", BRITISH JOURNAL OF HAEMATOLOGY, Bd. 106, Nr. 4, 1. September 1999 (1999-09-01), Seiten 898-904, XP55012028, ISSN: 0007-1048, DOI: 10.1046/j. 1365-2141.1999.01660.x**
• **HANNELORE HAUBELT ET AL: "Variables influencing Platelet Function Analyzer-100TM closure times in healthy individuals", BRITISH JOURNAL OF HAEMATOLOGY, Bd. 130, Nr. 5, 1. September 2005 (2005-09-01), Seiten 759-767, XP55012046, ISSN: 0007-1048, DOI: 10.1111/j. 1365-2141.2005.05680.x**

**Beschreibung**

Die Erfindung liegt auf dem Gebiet der Gerinnungsdiagnostik und betrifft in vitro-Verfahren zur Bestimmung der Thrombozytenfunktion mit Hilfe von Messzellen. Die Verfahren ermöglichen die Gewinnung von Messergebnissen, die unabhängig von dem verwendeten Messzellentyp miteinander vergleichbar sind.

Physiologische Vorgänge, die einerseits die Fluidität des Blutes im Gefäßsystem gewährleisten und andererseits durch die Bildung von Blutgerinnseln die Vermeidung von extravasalen Blutverlusten sicherstellen, werden unter dem Begriff Hämostase zusammengefasst. An der Regulation der Hämostase sind eine Vielzahl von Proteinfaktoren beteiligt sowie auch zelluläre Komponenten, wie z.B. die Thrombozyten (Blutplättchen). Im Falle einer Gefäßverletzung kommt es zuerst zur Anlagerung von Thrombozyten an das subendotheliale Kollagen. Diese Adhäsion wird durch Adhäsivproteine, wie den von-Willebrand-Faktor (VWF) vermittelt. Während des Adhäsionsvorgangs werden die Thrombozyten aktiviert und schütten Mediatoren aus ihren Granulae aus, wodurch die Aggregation weiterer Thrombozyten sowie eine Verstärkung der Aktivierung induziert wird. Auf diese Weise erfolgt ein primärer Gefäßwandverschluss (primäre Hämostase), der erst durch weitere Reaktionen des plasmatischen Gerinnungssystems (sekundäre Hämostase) stabilisiert wird. Fehlregulationen dieser Prozesse können zu einer Thromboseneigung oder einer Blutungsneigung führen und je nach Schweregrad lebensbedrohliche Folgen haben.

In der Gerinnungsdiagnostik sind verschiedene Verfahren und Systeme bekannt, mit deren Hilfe bestimmt werden kann, ob das Blut eines Patienten einwandfrei gerinnen kann oder ob eine Gerinnungsstörung vorliegt. Im Fall einer Gerinnungsstörung ist es häufig erforderlich, präzisere Kenntnisse über die Ursache der vorliegenden Störung zu erlangen, um die optimalen therapeutischen Maßnahmen auswählen zu können. Eine wichtige Teilfunktion des Gerinnungssystems, die gezielt untersucht werden kann, ist die primäre Hämostase, die wesentlich von der Funktionsfähigkeit der Thrombozyten abhängt.

Ein bekanntes Verfahren zur Untersuchung der Thrombozytenfunktion ist die Blutungszeitbestimmung. Es handelt sich dabei um einen in vivo-Globaltest, der die primäre Hämostase erfasst. Die Blutungszeit wird bestimmt, indem dem Patienten eine kleine Schnitt- oder Stichverletzung zugefügt wird und die Zeit bis zum Blutungsstillstand gemessen wird. Es handelt sich um einen schwer standardisierbaren, grob informativen Test, der vor allem in Notfallsituationen angewendet wird, um einen Überblick über die primäre Hämostase zu erhalten. Die Einnahme von Thrombozytenaggregationshemmern führt zu einer Verlängerung der Blutungszeit. Nachteilig an der Blutungszeitbestimmung ist, dass bei einer normalen Blutungszeit eine Thrombozytenfunktionsstörung nicht ausgeschlossen werden kann.

In vitro-Verfahren ermöglichen einen wesentlich empfindlicheren Nachweis von Thrombozytenfunktionsstörungen. Bei diesen Verfahren wird üblicherweise in einer Vollblutprobe oder in einer Probe von plättchenreichem Plasma (PRP) die Aggregation der Thrombozyten durch Zugabe eines Aktivators induziert und die Aggregationsreaktion gemessen. Die meistverwendeten Aktivatoren, die zur Induktion der Thrombozytenaggregation verwendet werden, sind: ADP (Adenosin-5'-diphosphat), Kollagen, Epinephrin (Adrenalin), Ristocetin und verschiedene Kombinationen davon sowie Thrombin, TRAP (Thrombin-Receptor-Activating-Protein), U-46619, Heparin (insbesondere im Fall von Heparininduzierter Thrombozytopenie) oder Serotonin.

Ein bekanntes System zur Bestimmung der Thrombozytenfunktion in vitro ist das sogenannte Platelet Function Analyzer System, kurz PFA-System (PFA-100®, PFA-200, Siemens Healthcare Diagnostics Products GmbH, Marburg, Deutschland). Mit Hilfe des PFA-Systems wird in Vollblutproben die primäre Hämostase unter Flussbedingungen und damit in Gegenwart von hohen Scherkräften gemessen.

Zur Simulation der Flussbedingungen und der Scherkräfte, wie sie in kleineren arteriellen Blutgefäßen herrschen, wird in einer PFA-Messzelle, die in PFA-Analysegerät eingesetzt wird, ein Unterdruck von etwa -40mbar erzeugt, und das Citratvollblut, dass sich in einem Probenreservoir befindet, strömt durch eine Kapillare, die einen Durchmesser von etwa 200 μm hat. Die Kapillare mündet in eine Messkammer, die mit einem Trennelement, z.B. einer Membran abgeschlossen ist, welche eine kapillare zentrale Öffnung (Apertur) enthält, durch die das Blut aufgrund des Unterdrucks durchtritt. In den meisten Fällen ist die Membran, zumindest in dem Bereich um die Apertur mit einem oder mehreren Aktivatoren, die die Thrombozytenaggregation induzieren, versetzt, so dass das vorbeiströmende Blut mit den aggregationsinduzierenden Substanzen im Bereich der Apertur in Kontakt kommt. Infolge der induzierten Adhäsion und Aggregation der Thrombozyten bildet sich im Bereich der Apertur ein Thrombozytenpfropf (Thrombus), der die Membranöffnung verschließt und den Blutfluss stoppt. In diesem System wird die Zeit gemessen, die bis zum Verschluss der Membranöffnung benötigt wird. Diese sogenannte Verschlusszeit korreliert mit der Funktionsfähigkeit der Thrombozyten. Eine Messzelle zur Verwendung in einem Verfahren zu Bestimmung der Thrombozytenfunktion anhand der Verschlusszeit ist z.B. in dem Patentdokument WO 97/34698 beschrieben. Bislang werden in dem Verfahren zur Bestimmung der Verschlusszeit Messzellen verwendet, die mit einer Membran ausgestattet sind, die mit Kollagen (Kol) und zusätzlich entweder mit ADP oder mit Epinephrin (Epi) beschichtet ist. Andere Messzellen, die insbesondere zur Bestimmung von Antithrombotika aus der Gruppe der P2Y(12)-Antagonisten, wie z.B. von Clopidogrel, geeignet sind, sind mit einer Membran ausgestattet, die ADP und Prostaglandin E1 enthält (INNOVANCE® PFA P2Y, EP-A1-1850134).

Ein bekanntes Verfahren zur Bestimmung der Thrombozytenfunktion in einer Probe mittels eines PFA-Systems, umfasst folgende Verfahrensschritte:

a) Verwendung einer Messzelle, wobei die Messzelle folgende folgende Elemente aufweist:

i) eine Haltekammer zum Halten der Probe,
ii) eine Kapillare, durch die die Probe von der Haltekammer in eine Messkammer geleitet wird,
iii) eine Messkammer, die durch ein Trennelement in zwei Kompartimente unterteilt ist, wobei das erste Kompartiment die Probe aus der Kapillare aufnimmt,
iv) ein Trennelement, das die Messkammer in zwei Kompartimente unterteilt und das eine Öffnung aufweist, durch welche die Probe aus dem ersten Kompartiment in das zweite Kompartiment fließen kann;

b) Befüllen der Haltekammer der Messzelle mit der Probe;
c) Platzieren der Messzelle in einem Gerät zur automatischen Bestimmung der Thrombozytenfunktion, wobei das Gerät folgende Elemente aufweist:

i) Mittel zum Anlegen eines Unterdrucks in der Messkammer der Messzelle,
ii) Mittel zur Bestimmung des Gesamtvolumens, welches durch das Anlegen eines Unterdrucks aus der Messzelle gezogen wird, wenn in der Messkammer der Messzelle ein Unterdruck angelegt ist;

d) Anlegen eines Unterdrucks in der Messkammer der Messzelle und Durchleiten der Probe durch die Kapillare und durch die Öffnung des Trennelements.

Das Gesamtvolumen, welches durch das Anlegen eines Unterdrucks aus der Messzelle gezogen wird, wird kontinuierlich bestimmt. Aus dem Volumen, welches pro Zeiteinheit aus der Messzelle gezogen wird, wird die Flussrate ($\mu$L/min) bestimmt.

Die Flussrate nimmt typischerweise über die Zeit ab, da der Thrombozytenpfropf die Apertur zunehmend verengt und so den Durchtritt der Probenflüssigkeit erschwert.

Als Testergebnis wird die Verschlusszeit in Sekunden ausgegeben. Die Verschlusszeit ist definiert als der Zeitpunkt, in dem die Flussrate für eine Dauer von drei Sekunden weniger als 10 % der initialen Flussrate betragen hat. Die initiale Flussrate ist die Flussrate zu Beginn der Messung, wenn sich an der Apertur noch kein Thrombozytenpfropf gebildet hat, aber das Totvolumen bereits aus dem System gezogen wurde.

Weicht die Verschlusszeit einer Patientenprobe vom Referenzbereich ab, weist dies auf eine Störung der Thrombozytenfunktion hin. Verlängerte Verschlusszeiten weisen auf das Vorliegen einer Thrombozytendysfunktion im Sinne einer verminderten Aggregationsfähigkeit hin. Verkürzte Verschlusszeiten weisen auf das Vorliegen einer Thrombozytendysfunktion im Sinne einer erhöhten Aggregationsfähigkeit hin.

Die Verschlusszeit einer Probe eines gesunden Spenders mit normaler Thrombozytenfunktion hängt von vielen Faktoren ab.

In erster Linie wird die Verschlusszeit beeinflusst durch die Art der verwendeten Messzelle. Wie oben erläutert, werden Messzellen verwendet, die verschiedene Kombinationen von Thrombozytenaktivatoren oder -inhibitoren enthalten. Bei Verwendung einer Messzelle (Kol/Epi), die Kollagen und Epinephrin enthält, liegt die Verschlusszeit einer Normalprobe zwischen 84-160 Sekunden. Bei Verwendung einer Messzelle (Kol/ADP), die Kollagen und ADP enthält, liegt die Verschlusszeit einer Normalprobe zwischen 68-121 Sekunden. Bei Verwendung einer Messzelle (ADP/PGE 1), die ADP und Prostaglandin E1 enthält, liegt die Verschlusszeit einer Normalprobe unter 106 Sekunden. Die starken Abweichungen der Referenzbereiche haben den Nachteil, dass eine Verschlusszeit als solche nicht aussagekräftig ist, sondern immer nur im Zusammenhang mit dem verwendeten Messzellentyp gedeutet werden kann. Eine Verschlusszeit von beispielsweise 130 Sekunden ist bei Kol/Epi-Messzellen ein normales Ergebnis, bei Kol/ADP- oder ADP/PGE1-Messzellen hingegen ein abnormales Ergebnis, das auf eine Blutungsneigung schließen lässt.

Ein weiterer Faktor, der die Verschlusszeit beeinflusst, ist die Messzellenarchitektur. Unterschiedliche Durchmesser der Apertur oder der Kapillare beeinflussen die Thrombusbildung und damit die Geschwindigkeit, mit der die Apertur verschlossen wird.

Es ist daher wünschenswert, das zuvor beschriebene bekannte Verfahren zur Bestimmung der Thrombozytenfunktion so zu verbessern, dass die Messung der Thrombozytenfunktion in einem PFA-System für alle verwendeten Messzellentypen vereinheitlicht und standardisiert werden kann, so dass die Messergebnisse direkt miteinander vergleichbar sind, unabhängig davon, welcher Messzellentyp verwendet wird.

Diese Aufgabe wird dadurch gelöst, dass das Probenvolumen, welches innerhalb eines definierten Zeitintervalls durch die Öffnung des Trennelements tritt, bestimmt wird und der bestimmte Probenvolumenwert dann mit einem Referenzprobenvolumenwert für normale Thrombozytenfunktion verglichen wird.

Die Bestimmung des Probenvolumens, welches innerhalb eines definierten Zeitintervalls durch die Öffnung des Trennelements tritt, erfolgt bevorzugterweise dadurch, dass das Gesamtvolumen, welches durch das Anlegen eines Unterdrucks aus der Messzelle gezogen wird, gemessen wird und die Differenz zwischen dem gemessenen Gesamtvolumen und dem Totvolumen der verwendeten Messzelle bestimmt wird.

Die Messung des Gesamtvolumens, welches durch das Anlegen eines Unterdrucks aus der Messzelle gezogen wird, wenn in der Messkammer der Messzelle ein Unterdruck angelegt ist, kann bekanntermaßen dadurch bestimmt werden, dass das Volumen gemessen wird, das das Mittel zum Anlegen eines Unterdrucks in der Messkammer der Messzelle aus der Messzelle zieht. Das Mittel zum Anlegen eines Unterdrucks besteht bevorzugterweise aus einem Zylinder mit einem Kolben, wobei der Kolben über einen Schrittmotor und eine Kolbenstange in axialer Richtung des Zylinders beweglich ist. Durch Bewegen des Kolbens wird ein Unterdruck in der Messzelle erzeugt. In der Messkammer der Messzelle bzw. in dem Raum, der durch Verbinden der Messzelle mit dem Zylinder geschaffen wird und in welchem der Unterdruck aufgebaut wird, misst ein Drucksensor den herrschenden Druck. In einer Steuervorrichtung wird der gemessene Druck mit einem vorgegebenen Drucksollwert verglichen. Beim Einfließen von Blut in die Kapillare steigt der Druck. Um den Druck konstant zu halten, z.B. auf -40 mbar, steuert die Steuervorrichtung den Schrittmotor, der den Kolben entsprechend axial bewegt. Über den zurückgelegten Weg des Kolbens in dem Zylinder mit bekannten Durchmesser kann das Gesamtvolumen, welches aus der Messzelle gezogen wird, berechnet werden.

Das Gesamtvolumen, welches aus der Messzelle gezogen wird, setzt sich aus dem Totvolumen der Messzelle und dem Volumen der tatsächlich durch die Öffnung des Trennelements getretenen Probe zusammen (Probenvolumen). Die Differenz zwischen gemessenem Gesamtvolumen und dem bekannten messzellenspezifischen Totvolumen ergibt das Probenvolumen, welches innerhalb eines definierten Zeitintervalls oder bis zum Verschluss der Apertur durch die Öffnung des Trennelements tritt.

Das Totvolumen einer Messzelle entspricht dem Volumen, das, wenn in der Messkammer der Messzelle ein Unterdruck angelegt ist, durch die Öffnung des Trennelements tritt bevor erstmalig Probenflüssigkeit das Trennelement durchtritt. Das Totvolumen einer Messzelle besteht üblicherweise aus Luft, die insbesondere in der Kapillare der Messzelle und in dem vor dem Trennelement gelegenen Kompartiment der Messkammer, welches die Probe aus der Kapillare aufnimmt, enthalten ist.

Das Totvolumen ist abhängig von der Messzellenarchitektur. Das Totvolumen eines speziellen Messzellentyps kann entweder berechnet werden, oder es wird vorab experimentell bestimmt. Zur experimentellen Bestimmung wird bevorzugterweise das Totvolumen mehrerer gleichartiger Messzellen bestimmt und der Median der gemessenen Totvolumina wird als Totvolumen für Messzellen dieses Typs angewandt.

Alternativ kann die Bestimmung des Probenvolumens, welches innerhalb eines definierten Zeitintervalls durch die Öffnung des Trennelements tritt, dadurch erfolgen, dass der Füllstand des Probenvolumens im zweiten Kompartiment der Messkammer elektrisch oder optisch bestimmt wird.

Der bestimmte Probenvolumenwert wird dann mit einem Referenzprobenvolumenwert für normale Thrombozytenfunktion verglichen.

Der Referenzprobenvolumenwert für normale Thrombozytenfunktion ist abhängig von dem verwendeten Messzellentyp, und er wird vorab experimentell bestimmt. Zur experimentellen Bestimmung wird das Probenvolumen, welches innerhalb eines definierten Zeitintervalls oder bis zum Verschluss der Apertur durch die Öffnung des Trennelements tritt, einer hinreichend großen Anzahl von Proben offensichtlich gesunder Blutspender (Normalproben) in Messzellen desselben Typs bestimmt, und der Median der gemessenen Probenvolumina wird als Referenzprobenvolumenwert für Messzellen dieses Typs angewandt.

Bevorzugterweise wird das bestimmte Probenvolumen zu einem Referenzprobenvolumenwert für normale Thrombozytenfunktion ins Verhältnis gesetzt. Besonders bevorzugt beträgt dabei der Referenzprobenvolumenwert für normale Thrombozytenfunktion 100 %. Ein so ermitteltes Testergebnis ist ein Maß für die Thrombozytenfunktion im Vergleich zur Norm (% der Norm). Es kann auch als Primäre Hämostase Kapazität 1 bezeichnet (PHC 1) werden.

Eine Formel zur Berechnung lautet zum Beispiel ("Formel (1) ") :

$$PHC(1)\ \%\ =\ \frac{(TV_{median}\ -\ DV_{median})\ \times\ 100}{(TV\ -\ DV_{median})}$$

mit

$TV_{median}$ = Median des Gesamtvolumenwerts für normale Thrombozytenfunktion,

$DV_{median}$ = Median des Totvolumens des verwendeten Messzellentyps,

$TV$ = Gesamtvolumen der zu analysierenden Probe.

Die erfindungsgemäße Aufgabe wird auch dadurch gelöst, dass das Probenvolumen, welches innerhalb eines definierten Zeitintervalls durch die Öffnung des Trennelements tritt, bestimmt wird und dass die initiale Flussrate bestimmt wird, und dass dann die Differenz von dem Fünffachen der initialen Flussrate und dem Probenvolumen gebildet wird und die Differenz dann mit einem Referenzdifferenzwert für normale Thrombozytenfunktion verglichen wird.

Die Bestimmung des Probenvolumens erfolgt wie bereits oben beschrieben.

Die initiale Flussrate ist die maximale Flussrate der Probenflüssigkeit zu Beginn der Messung. Die Bestimmung der initialen Flussrate erfolgt bevorzugterweise dadurch, dass die Flussrate, d.h. das Volumen, das pro Zeiteinheit aus der Messzelle gezogen wird, ab dem Beginn der Messung, d.h. ab dem Zeitpunkt, in dem ein Unterdruck in der Messkammer der Messzelle angelegt wird, kontinuierlich bestimmt wird. Ganz zu Beginn der Messung kommt es unter anderem wegen des zunächst angesaugten Totvolumens zu gewissen Schwankungen in der Flussrate bis sich schließlich eine kontinuierlich abnehmende Flussrate einstellt. Die initiale Flussrate ist definiert als die Flussrate, die gemessen wurde bevor die Flussrate schließlich für mindestens 10 Sekunden kontinuierlich abnimmt.

Es wird die Differenz von dem Fünffachen der bestimmten initialen Flussrate und dem bestimmten Probenvolumen gebildet, und die Differenz wird dann mit einem Referenzdifferenzwert für normale Thrombozytenfunktion verglichen.

Der Referenzdifferenzwert wird für jede Probenmessung individuell bestimmt, indem die Differenz von dem Fünffachen der gemessenen, probenspezifischen initialen Flussrate und dem vorab bestimmten Referenzprobenvolumenwert für normale Thrombozytenfunktion gebildet wird. Die Bestimmung des Referenzprobenvolumenwerts für normale Thrombozytenfunktion erfolgt wie bereits oben beschrieben.

Bevorzugterweise wird die bestimmte Differenz von dem Fünffachen der bestimmten initialen Flussrate und dem bestimmten Probenvolumen zu einem Referenzdifferenzwert für normale Thrombozytenfunktion ins Verhältnis gesetzt. Besonders bevorzugt beträgt dabei der Referenzdifferenzwert für normale Thrombozytenfunktion 100 %. Ein so ermitteltes Testergebnis ist ein Maß für die Thrombozytenfunktion im Vergleich zur Norm (% der Norm). Es kann auch als Primäre Hämostase Kapazität 2 bezeichnet (PHC 2) werden.

Eine Formel zur Berechnung lautet zum Beispiel ("Formel (2) ") :

$$PHC(2)\ \% = \frac{(5\ min\ \times\ IF\ -\ (TV\ -\ DV_{median}))\ \times\ 100}{5\ min\ \times\ IF\ -\ (TV_{median}\ -\ DV_{median})}$$

mit

IF = initiale Flussrate der zu analysierenden Probe,
TVmedian = Median des Gesamtvolumenwerts für normale Thrombozytenfunktion,
DVmedian = Median des Totvolumens des verwendeten Messzellentyps,
TV = Gesamtvolumen der zu analysierenden Probe.

Der Vorteil der vorliegenden Erfindung besteht darin, dass, unabhängig von der Art der verwendeten Messzelle, d.h. unabhängig von der Art der verwendeten Thrombozytenaktivierenden oder inhibierenden Substanzen und unabhängig von der Messzellenarchitektur, Proben mit erniedrigter Thrombozytenfunktion (Blutungsrisiko) immer Testergebnisse von kleiner als 100 % aufweisen und Proben mit erhöhter Thrombozytenfunktion (Thromboserisiko) immer Testergebnisse von größer als 100 % aufweisen. Die Normalbereiche der verschiedenen Messzellentypen unterscheiden sich aufgrund dieser Standardisierung kaum.

## FIGURENBESCHREIBUNG

### Figur 1

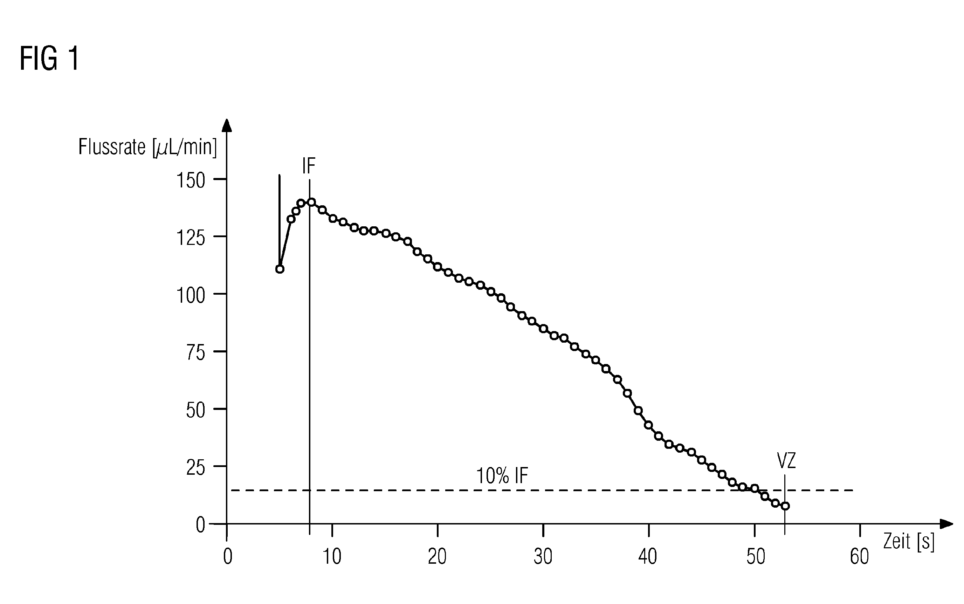

Figur 1 zeigt eine typische Flusskurve einer Vollblutprobe eines gesunden Spenders mit normaler Thrombozytenaktivität in einer INNOVANCE PFA P2Y-Messzelle. Nach anfänglichen Schwankungen der Flussrate zu Beginn der Messung, stellt sich eine kontinuierlich abnehmende Flussrate ein. Die Flussrate, die am Beginn der kontinuierlichen Abnahme gemessen wurde, stellt die initiale Flussrate (IF) dar. Die Verschlusszeit (VZ) ist definiert als der Zeitpunkt, in dem die Flussrate für eine Dauer von drei Sekunden weniger als 10 % der initialen Flussrate betragen hat.

### Figur 2

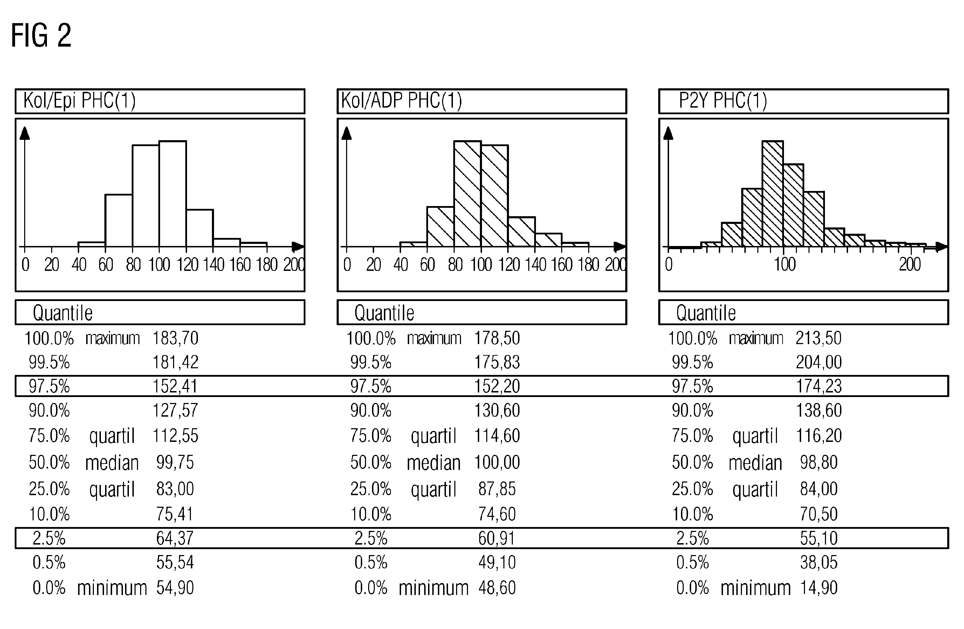

Figur 2 zeigt die Verteilung und Perzentile der PHC(1)-Werte von Bestimmungen der Thrombozytenaktivität in Vollblutproben gesunder Spender (N = 138 gesunde Blutspender) mit Kol/Epi-, Kol/ADP- und INNOVANCE PFA P2Y- (P2Y)-Messzellen. Es zeigt sich, dass die Ober- und Untergrenzen der 95 %igen Zentralintervalle von PHC(1) aller drei Messzellentypen eng beieinander liegen.

**Figur 3**

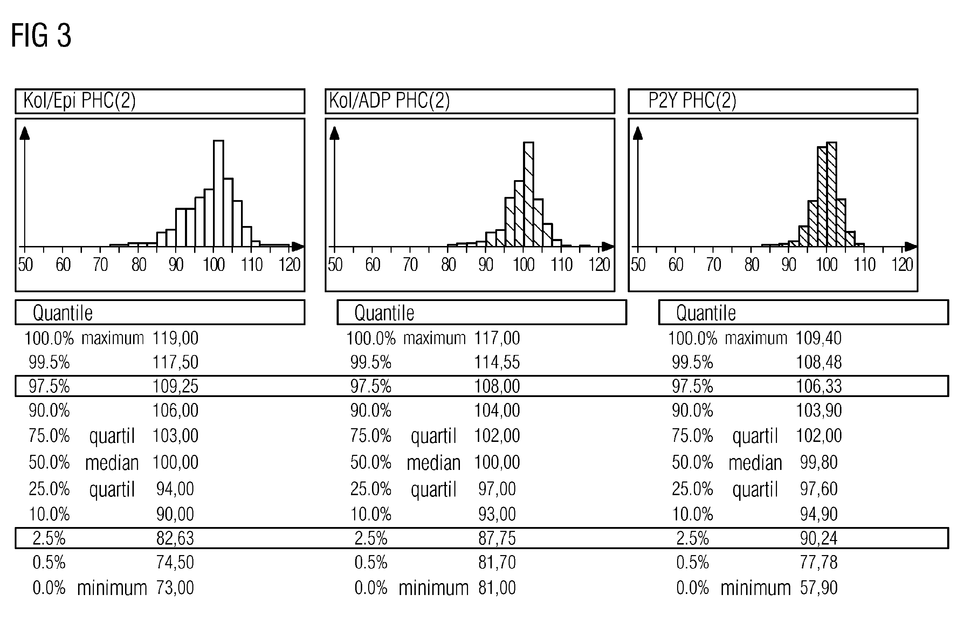

Figur 3 zeigt die Verteilung und Perzentile der PHC(2)-Werte von Bestimmungen der Thrombozytenaktivität in Vollblutproben gesunder Spender (N = 138 gesunde Blutspender) mit Kol/Epi-, Kol/ADP- und INNOVANCE PFA P2Y- (P2Y)-Messzellen. Auch hier zeigt sich, dass die Ober- und Untergrenzen der 95 %igen Zentralintervalle von PHC(2) aller drei Messzellentypen eng beieinander liegen.

**Figur 4**

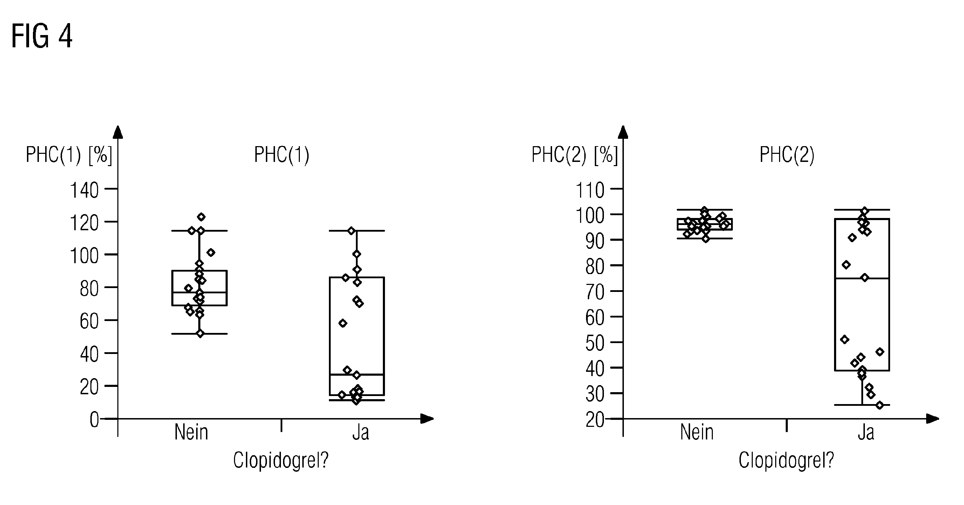

Figur 4 zeigt die Verteilung der PHC(1)- und PHC(2)-Werte von Bestimmungen der Thrombozytenaktivität in Vollblutproben bei kardiologischen Patienten vor (N = 23) und nach (N = 23) Clopidogreleinnahme mit INNOVANCE PFA P2Y-Messzellen. Es zeigt sich, dass sowohl PHC(1) als auch PHC(2) auch bei stark reduzierter Thrombozytenfunktion differenzierbare Ergebnisse liefern.

**BEISPIELE**

Die folgenden Beispiele veranschaulichen die Erfindung und sind nicht als Einschränkung zu verstehen.

**BEISPIEL 1: Bestimmung der Thrombozytenfunktion in % der Norm anhand von PHC(1) und PHC(2) in Proben gesunder Spender**

Blutproben von 138 gesunden Blutspendern in 3,2 %igem und 3,8 %igem gepuffertem Natriumzitrat wurden mit drei verschiedenen PFA Messzellentypen in einem PFA-Gerät (PFA-100, Siemens Healthcare Diagnostics Products GmbH) in Doppelbestimmung gemessen. Es wurden Messzellen verwendet, die mit einer Membran ausgestattet sind, die mit Kollagen (Kol) und mit ADP beschichtet ist (Kol/ADP), oder die mit Kollagen (Kol) und mit Epinephrin (Epi) beschichtet ist (Kol/Epi), oder die mit ADP und Prostaglandin E1 beschichtet ist (INNOVANCE® PFA P2Y, kurz: P2Y). Aus den vom Gerät ermittelten Rohdaten wurden das Gesamtvolumen, das Totvolumen (Luftvolumen, das während der ersten vier Sekunden der Messung aus der Messzelle gezogen wird) und die initiale Flussrate ermittelt. Vom Gesamtvolumen und vom Totvolumen aller Messungen (vier Messungen mit P2Y-Messzellen bzw. zwei Messungen mit Kol/Epi- und Kol/ADP-Messzellen je Spender) wurde daraufhin der Median des Gesamtvolumenwertes für normale Thrombozytenfunktion $TV_{median}$, also der Referenzprobenvolumenwert für normale Thrombozytenfunktion, und der Median des Totvolumens des verwendeten Messzellentyps $DV_{median}$ berechnet. Die Medianwerte sind in Tabelle 1 dargestellt.

**Tabelle 1**

| Messzellentyp | $TV_{median}$ [$\mu$l] | $DV_{median}$ [$\mu$l] |
|---|---|---|
| **Kol/ADP** | 300 | 159 |
| **Kol/Epi** | 350 | 159 |
| **P2Y** | 223 | 144 |

Diese Medianwerte wurden in den oben aufgeführten Formeln (1) und (2) als Konstanten zur Berechnung der Thrombozytenfunktion in % der Norm jeder Probe verwendet.

Die Verteilung der normalisierten PHC(1)-Werte ist in Figur 2 gezeigt. Die Verteilung der PHC(2)-Werte ist in Figur 3 gezeigt.

Es zeigt sich, dass die Ober- und Untergrenzen der 95 %igen Zentralintervalle von PHC(1) und PHC(2) der drei Messzellentypen recht eng beieinander liegen. Die kleinen Unterschiede sind auf die Messzellencharakteristik, z. B. die Stärke und Konzentration der verwendeten Thrombozytenagonisten, zurückzuführen.

Mit dem erfindungsgemäßen Verfahren bestimmte Ergebnisse für die Thrombozytenfunktion sind direkt miteinander vergleichbar, unabhängig davon welcher Messzellentyp verwendet wird.

**Beispiel 2: Bestimmung der Thrombozytenfunktion in % der Norm anhand von PHC(1) und PHC(2) in Proben von antikoagulierten Patienten**

Der Einfluss der Einnahme von Clopidogrel, einem Inhibitor der Thrombozytenfunktion, auf die PHC(1)- und PHC(2)-Werte von INNOVANCE PFA P2Y Messungen wurde anhand von kardiologischen Patienten untersuchten. Dazu wurde den Patienten jeweils vor und mindestens 4 Stunden nach Einnahme von Clopidogrel (300 mg) in 3,2 % gepuffertem Natriumzitrat Blut abgenommen, und die Thrombozytenaktivität wurde mit INNOVANCE PFA P2Y Messzellen gemessen. Zur Berechnung der Thrombozytenfunktion in % der Norm jeder Probe wurden die Medianwerte wie in Tabelle 1 gezeigt verwendet. In Figur 4 sind die PHC(1)- und PHC(2)-Werte vor und nach Clopidogreleinnahme aufgezeigt.

Es zeigt sich dabei, dass sowohl PHC(1) als auch PHC(2) auch bei stark reduzierter Thrombozytenfunktion differenzierbare Ergebnisse liefern, was bei der üblicherweise verwendeten Verschlusszeit nicht der Fall ist, da dann alle Ergebnisse als >300 s berichtet werden.

**Patentansprüche**

1.  Verfahren zur Bestimmung der Thrombozytenfunktion in einer Probe, wobei das Verfahren folgende Schritte umfasst:

    a) Verwendung einer Messzelle, wobei die Messzelle folgende Elemente aufweist:

    i) eine Haltekammer zum Halten der Probe,
    ii) eine Kapillare, durch die die Probe von der Haltekammer in eine Messkammer geleitet wird,
    iii) eine Messkammer, die durch ein Trennelement in zwei Kompartimente unterteilt ist, wobei das erste Kompartiment die Probe aus der Kapillare aufnimmt,
    iv) ein Trennelement, das die Messkammer in zwei Kompartimente unterteilt und das eine Öffnung aufweist, durch welche die Probe aus dem ersten Kompartiment in das zweite Kompartiment fließen kann;

    b) Befüllen der Haltekammer der Messzelle mit der Probe;
    c) Platzieren der Messzelle in einem Gerät zur automatischen Bestimmung der Thrombozytenfunktion, wobei das Gerät folgende Elemente aufweist:

    i) Mittel zum Anlegen eines Unterdrucks in der Messkammer der Messzelle,
    ii) Mittel zur Bestimmung des Gesamtvolumens, welches durch das Anlegen eines Unterdrucks aus der Messzelle gezogen wird, wenn in der Messkammer der Messzelle ein Unterdruck angelegt ist;

    d) Anlegen eines Unterdrucks in der Messkammer der Messzelle und Durchleiten der Probe durch die Kapillare und durch die Öffnung des Trennelements;
    **dadurch gekennzeichnet, dass**
    das Verfahren ferner folgende Schritte umfasst:
    e) Bestimmen des Probenvolumens, welches innerhalb eines definierten Zeitintervalls durch die Öffnung des Trennelements tritt, und
    f) Vergleich des bestimmten Probenvolumens mit einem Referenzprobenvolumenwert für normale Thrombozytenfunktion;
    oder
    g) Bestimmen des Probenvolumens, welches innerhalb eines definierten Zeitintervalls durch die Öffnung des Trennelements tritt, und
    h) Bestimmen der initialen Flussrate, und
    i) Bilden der Differenz von dem Fünffachen der initialen Flussrate und dem Probenvolumen und Vergleich der Differenz mit einem Referenzdifferenzwert für normale Thrombozytenfunktion.

2.  Verfahren nach Anspruch 1, wobei in Schritt e) oder in Schritt g) die Bestimmung des Probenvolumens, welches innerhalb eines definierten Zeitintervalls durch die Öffnung des Trennelements tritt, dadurch erfolgt, dass das Gesamtvolumen, welches durch das Anlegen eines Unterdrucks aus der Messzelle gezogen wird, gemessen wird und die Differenz zwischen dem gemessenen Gesamtvolumen und dem Totvolumen der verwendeten Messzelle bestimmt wird.

3.  Verfahren nach Anspruch 1, wobei in Schritt f) das bestimmte Probenvolumen zu einem Referenzprobenvolumenwert für normale Thrombozytenfunktion ins Verhältnis gesetzt wird.

4.  Verfahren nach Anspruch 3, wobei der Referenzprobenvolumenwert für normale Thrombozytenfunktion 100 % beträgt.

5. Verfahren nach Anspruch 1, wobei in Schritt i) die gebildete Differenz zu einem Referenzdifferenzwert für normale Thrombozytenfunktion ins Verhältnis gesetzt wird.

6. Verfahren nach Anspruch 5, wobei der Referenzdifferenzwert für normale Thrombozytenfunktion 100 % beträgt.

**Claims**

1. Method for determining thrombocyte function in a sample, wherein the method comprises the following steps:

   a) using a measuring cell, wherein the measuring cell comprises the following elements:

      i) a retention chamber for retaining the sample,
      ii) a capillary through which the sample is conducted from the retention chamber into a measuring chamber,
      iii) a measuring chamber which is divided by a partition element into two compartments, wherein the first compartment accommodates the sample from the capillary,
      iv) a partition element which divides the measuring chamber into two compartments and which has an aperture through which the sample can flow from the first compartment into the second compartment;

   b) filling the retention chamber of the measuring cell with the sample;
   c) placing the measuring cell in a device for automatic determination of thrombocyte function, wherein the device comprises the following elements:

      i) means for applying negative pressure in the measuring chamber of the measuring cell,
      ii) means for determining the total volume which, as a result of the application of negative pressure, is drawn from the measuring cell when negative pressure is applied in the measuring chamber of the measuring cell;

   d) applying negative pressure in the measuring chamber of the measuring cell and conducting the sample through the capillary and through the aperture in the partition element;
   **characterised in that**
   the method further comprises the following steps:
   e) determining the sample volume passing within a defined time interval through the aperture in the partition element, and
   f) comparing the determined sample volume with a reference sample volume value for normal thrombocyte function;
   or
   g) determining the sample volume passing within a defined time interval through the aperture in the partition element, and
   h) determining the initial flow rate, and
   i) calculating the difference between five times the initial flow rate and the sample volume and comparing the difference with a reference difference value for normal thrombocyte function.

2. Method according to Claim 1, wherein, in step e) or in step g), the sample volume passing through the aperture in the partition element within a defined time interval is determined by measuring the total volume which, as a result of the application of negative pressure, is drawn from the measuring cell and determining the difference between the measured total volume and the dead volume of the measuring cell used.

3. Method according to Claim 1, wherein, in step f), the determined sample volume is expressed in relation to a reference sample volume value for normal thrombocyte function.

4. Method according to Claim 3, wherein the reference sample volume value for normal thrombocyte function is 100%.

5. Method according to Claim 1, wherein, in step i), the calculated difference is expressed in relation to a reference difference value for normal thrombocyte function.

6. Method according to Claim 5, wherein the reference difference value for normal thrombocyte function is 100%.

**Revendications**

1. Procédé de détermination de la fonction de thrombocytes dans un échantillon, le procédé comprenant les stades suivants :

   a) on utilise une cellule de mesure, la cellule de mesure ayant les éléments suivants :

      i) une chambre de retenue pour retenir l'échantillon,
      ii) un tube capillaire, par lequel l'échantillon passe de la chambre de retenue à une chambre de mesure,
      iii) une chambre de mesure, qui est subdivisée par un élément de séparation en deux compartiments, le premier compartiment recevant l'échantillon du tube capillaire,
      iv) un élément de séparation, qui subdivise la chambre de mesure en deux compartiments et qui a une ouverture, par laquelle l'échantillon peut s'écouler du premier compartiment au deuxième compartiment,

   b) on remplit de l'échantillon la chambre de retenue de la cellule de mesure ;
   c) on met la cellule de mesure dans un appareil de détermination automatique de la fonction de thrombocytes, l'appareil ayant les éléments suivants :

      i) des moyens d'application d'une dépression dans la chambre de mesure de la cellule de mesure,
      ii) des moyens de détermination du volume total, qui, par l'application d'une dépression, est extrait de la cellule de mesure, lorsqu'une dépression est appliquée dans la chambre de mesure de la cellule de mesure,

   d) on applique une dépression dans la chambre de mesure de la cellule de mesure et on fait passer l'échantillon dans le tube capillaire et dans l'ouverture de l'élément de séparation ;
   **caractérisé en ce que**
   le procédé comprend, en outre, les stades suivants :
   e) on détermine le volume d'échantillon, qui, dans un intervalle de temps défini, entre dans l'ouverture de l'élément de séparation, et
   f) on compare le volume d'échantillon déterminé à une valeur de référence de volume d'échantillon pour une fonction de thrombocytes normale ;
   ou
   g) on détermine le volume d'échantillon, qui, dans un intervalle de temps défini, entre dans l'ouverture de l'élément de séparation, et
   h) on détermine le débit initial, et
   i) on forme la différence entre le quintuple du débit initial et le volume d'échantillon et on compare la différence à une valeur de différence de référence pour une fonction de thrombocytes normale.

2. Procédé suivant la revendication 1, dans lequel, dans le stade e) ou dans le stade g), on effectue la détermination du volume d'échantillon, qui, dans un intervalle de temps défini, entre dans l'ouverture de l'élément de séparation, en mesurant le volume total, qui, par l'application d'une dépression, est extrait de la cellule de mesure, et en déterminant la différence entre le volume total mesuré et le volume mort de la cellule de mesure utilisé.

3. Procédé suivant la revendication 1, dans lequel, dans le stade f), on met en relation le volume d'échantillon déterminé et une valeur de référence du volume d'échantillon pour une fonction de thrombocytes normale.

4. Procédé suivant la revendication 3, dans lequel la valeur de référence du volume d'échantillon pour une fonction de thrombocytes normale est de 100%.

5. Procédé suivant la revendication 1, dans lequel, dans le stade i), on met en relation la différence formée et une valeur de référence de différence pour une fonction de thrombocytes normale.

6. Procédé suivant la revendication 5, dans lequel la valeur de référence de différence pour une fonction de thrombocytes normale est de 100%.

FIG 1

Flussrate [μL/min]

150
125
100
75
50
25
0

IF

10% IF

VZ

0    10    20    30    40    50    60    Zeit [s]

EP 2 543 998 B1

## FIG 2

**Kol/Epi PHC(1)**

Quantile

| 100.0% | maximum | 183,70 |
|---|---|---|
| 99.5% | | 181,42 |
| 97.5% | | 152,41 |
| 90.0% | | 127,57 |
| 75.0% | quartil | 112,55 |
| 50.0% | median | 99,75 |
| 25.0% | quartil | 83,00 |
| 10.0% | | 75,41 |
| 2.5% | | 64,37 |
| 0.5% | | 55,54 |
| 0.0% | minimum | 54,90 |

**Kol/ADP PHC(1)**

Quantile

| 100.0% | maximum | 178,50 |
|---|---|---|
| 99.5% | | 175,83 |
| 97.5% | | 152,20 |
| 90.0% | | 130,60 |
| 75.0% | quartil | 114,60 |
| 50.0% | median | 100,00 |
| 25.0% | quartil | 87,85 |
| 10.0% | | 74,60 |
| 2.5% | | 60,91 |
| 0.5% | | 49,10 |
| 0.0% | minimum | 48,60 |

**P2Y PHC(1)**

Quantile

| 100.0% | maximum | 213,50 |
|---|---|---|
| 99.5% | | 204,00 |
| 97.5% | | 174,23 |
| 90.0% | | 138,60 |
| 75.0% | quartil | 116,20 |
| 50.0% | median | 98,80 |
| 25.0% | quartil | 84,00 |
| 10.0% | | 70,50 |
| 2.5% | | 55,10 |
| 0.5% | | 38,05 |
| 0.0% | minimum | 14,90 |

# FIG 3

**Kol/Epi PHC(2)**

Quantile

| | | |
|---|---|---|
| 100.0% | maximum | 119,00 |
| 99.5% | | 117,50 |
| 97.5% | | 109,25 |
| 90.0% | | 106,00 |
| 75.0% | quartil | 103,00 |
| 50.0% | median | 100,00 |
| 25.0% | quartil | 94,00 |
| 10.0% | | 90,00 |
| 2.5% | | 82,63 |
| 0.5% | | 74,50 |
| 0.0% | minimum | 73,00 |

**Kol/ADP PHC(2)**

Quantile

| | | |
|---|---|---|
| 100.0% | maximum | 117,00 |
| 99.5% | | 114,55 |
| 97.5% | | 108,00 |
| 90.0% | | 104,00 |
| 75.0% | quartil | 102,00 |
| 50.0% | median | 100,00 |
| 25.0% | quartil | 97,00 |
| 10.0% | | 93,00 |
| 2.5% | | 87,75 |
| 0.5% | | 81,70 |
| 0.0% | minimum | 81,00 |

**P2Y PHC(2)**

Quantile

| | | |
|---|---|---|
| 100.0% | maximum | 109,40 |
| 99.5% | | 108,48 |
| 97.5% | | 106,33 |
| 90.0% | | 103,90 |
| 75.0% | quartil | 102,00 |
| 50.0% | median | 99,80 |
| 25.0% | quartil | 97,60 |
| 10.0% | | 94,90 |
| 2.5% | | 90,24 |
| 0.5% | | 77,78 |
| 0.0% | minimum | 57,90 |

FIG 4

EP 2 543 998 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9734698 A
- EP 1850134 A1